# EUROPEAN PATENT SPECIFICATION

(11) **EP 0 726 763 B1**
(45) Date of publication and mention of the grant of the patent: **29.03.2000**
(21) Application number: 95900504.2
(22) Date of filing: 03.11.1994
(51) Int. Cl.: A61K 9/127, A61K 31/557

(54) **METHODS OF TREATMENT USING UNILAMELLAR LIPOSOMAL ARACHIDONIC ACID METABOLITE FORMULATIONS**
BEHANDLUNGSVERFAHREN UNTER VERWENDUNG UNILAMELLAR-LIPOSOMALER ARACHIDDONSÄUREMETABOLIT-FORMULIERUNGEN
PROCEDES DE TRAITEMENT FAISANT APPEL A DES FORMULATIONS DE METABOLITE D'ACIDE ARACHIDONIQUE LIPOSOMIQUE UNILAMELLAIRE

(30) Priority: 04.11.1993 US 147898; 16.11.1993 US 152852; 11.01.1994 US 180089
(43) Date of publication of application: 21.08.1996
(73) Proprietor: THE LIPOSOME COMPANY, INC., Princeton, NJ 08540 (US)
(72) Inventor: OSTRO, Marc J., Pennington, NJ 98534 (US); JANOFF, Andrew S., Yardley, PA 19067 (US); MINCHEY, Sharma R., Monmouth Junction, NJ 08852 (US)
(74) Representative: Weiss, Wolfgang, Dipl.-Chem. Dr.
(86) International application number: US9412579
(87) International publication number: WO9512388

(56) References cited:
- EP-A- 0 292 403
- EP-A- 0 416 527
- EP-A- 0 512 916
- DATABASE WPI Week 9304, Derwent Publications Ltd., London, GB; AN 93-030483 & JP,A,4 356 421 (GREEN CROSS CORP) 10 December 1992

## Description

This application is a continuation-in-part of U.S. Serial No. 08/152,852, filed November 16, 1993, which-in-turn is a continuation-in-part of U.S. Serial No. 07/821,648, filed November 16, 1992, now U.S. Patent No. 5,262,168, which-in-turn is a continuation of U.S. Serial No. 07/195,228, filed May 18, 1988, now U.S. Patent No. 5,082,664, which-in-turn is a continuation-in-part of U.S. Serial No. 053,305, filed May 2, 1987, now abandoned, and this application is also a continuation-in-part of U.S. Serial No. 08/180,089, filed January 11, 1994, which-in-turn is a continuation-in-part of U.S. Serial No. 147,898, filed November 4, 1993, now abandoned. The application is directed to the therapeutic uses of unilamellar liposomal arachidonic acid metabolite formulations.

The various prostaglandins are grouped into several categories (A-l), which are distinguished by varying substituents on the five-carbon ring introduced into the twenty-carbon fatty acid precursor during prostaglandin synthesis. These groups can be further subdivided based upon the number, and position, of double bonds in the prostaglandins' carbon chains. Prostaglandins are believed to act on their target cells by way of cellular surface receptors; these receptors are believed to be coupled to second messenger systems by which prostaglandin action is mediated. Prostaglandins can have a broad spectrum of biological activities.

Enzymes in the body can rapidly deactivate prostaglandins. This typically necessitates frequent administrations of high doses of the compounds to maintain therapeutically effective levels in the serum, thereby increasing the expense of prostaglandin treatment and leading to the possibility of unwanted side effects. Furthermore, as prostaglandin deactivation occurs primarily as blood passes through the lungs, the compounds are generally administered intra-arterially. Liposomal formulations can prolong the circulatory half-lives of arachidonic acid metabolites, e.g., prostaglandins, and can help avoid their deactivation in the lungs. Accordingly, such liposomal formulations can useful provide therapeutic alternatives.

Mizishuma et at. (J. Rheumatol. 14:97 (1987)) and Hoshi et at. (Drugs. Exptl. Clin. Res. 12(8):681 (1986)) describe lipid microspheres containing prostaglandin E₁ (PGE₁). However, as disclosed in Mizishuma et al. (U.S. Patent No. 4,493,847) and Imagawa et al. (U.S. Patent No. 4,684,633), these "microspheres" are actually prostaglandin-containing fat emulsions, which are not liposomes, and have neither the same properties, nor the same advantages, as the liposomal arachidonic acid metabolite formulations provided herein. Shell and See (U.S. Patent Nos. 4,820,732 and 4,955,878) disclose treatments for reducing dysfunction during angioplasty procedures which involve administering prostaglandin-containing compositions to patients. These compositions also contain a carrier. However, the liquid carriers disclosed, e.g., dehydrated alcohols and saline solutions, generally cannot provide sustained release of an arachidonic acid metabolite. The fat-laden microsphere carriers disclosed are taught to be at least as large as a red blood cell, i.e. at least 7 microns in diameter, and can be much larger. Administration of particles of such large size to animals can cause difficulties because the microspheres can become stuck in, and clog, small blood vessels, e.g., lung capillaries. The liposomes used in this invention, by contrast have a maximum size of about 5 microns or less, and are preferably about 50 nm to about 1 micron in size. These liposomes can safely be administered to animals for therapeutic purposes.

EP 0 416 527 describes prostaglandin-containing liposome preparations which are characterized in that positively charged lipid(s) is (are) incorporated in the lipid membrane. However, the necessity of introducing positively charged lipid bears the disadvantage that the latter are not suitable for certain medical applications. Here, a phosphate buffer is disclosed which clearly is not a release-inhibiting buffer.

Liposomal formulations of drugs can have an enhanced therapeutic index by reducing the drugs toxicity, increasing its efficacy, or both. The liposomal arachidonic acid metabolite formulations employed in the practice of this invention are useful in ameliorating or preventing diseases, disorders or conditions such as toxemic disorders, inflammatory disorders and cell activation and adhesion disorders.

This invention provides a method for the manufacture of a pharmaceutical composition for the treatment of a disorder characterized by cell activation and adhesion, inflammation or toxemia, comprising formulating a composition comprising a pharmaceutically acceptable carrier and a unilamellar liposome comprising (i) a lipid, (ii) a release-inhibiting aqueous buffer, and (iii) an anti-disorder effective amount of an arachidonic metabolite which is at least about 10⁻¹² g of the metabolite per kg of body weight of the animal to be treated, wherein said release-inhibiting buffer increases the strength of metabolite-lipid interactions or establishes electrostatic repulsions with the metabolite. The composition is suitable for administration to an animal. Preferably, the animal is a human and the administration comprises intravenous administration.

Further, the invention provides the use of a composition comprising a pharmaceutically acceptable carrier and a unilamellar liposome comprising an arachidonic acid metabolite, a lipid and a release-inhibiting aqueous buffer wherein said release-inhibiting buffer increases the strength of metabolite-lipid interactions or establishes electrostatic repulsions with the metabolite for the manufacture of a composition for the treatment of an animal afflicted with a disorder characterized by cell activation and adhesion, inflammation or toxemia.

Preferably, the unilamellar liposome is a large unilamellar liposome (LUV), more preferably, an LUV having a diameter of about 100 nm. Preferably, the arachidonic acid metabolite administered to the animal is a prostaglandin, more preferably a prostaglandin of the E or l series, and most preferably, prostaglandin E1. Preferably, the lipid is a saturated acyl chain lipid, more preferably, dipalmitoyl phosphatidylcholine (DPPC). Preferably, the buffer is a citric acid buffer, more preferably, a citric acid buffer having a pH of about 4.5.

The method of this invention can be used to treat animals afflicted with disorders characterized by cell activation and adhesion, inflammation or toxemia, amongst other indications. Such disorders include, without limitation: reperfusion injury, myocardial infarction, vaso-occlusive disease, adult respiratory distress syndrome (ARDS), systemic inflammatory response syndrome (SIRS), vasculitis, post-traumatic shock, burn injury, vaso-occlusive disorders, arthritic disorders, for example, gout, rheumatoid arthritis or filary attires, and autoimmune disorders, for example, systemic lupus erythematosus, juvenile diabetes, multiple sclerosis or Hashimoto's thyroiditis. Particularly preferred indications are ARDS and SIRS.

The method comprises administering to the animal an amount of the liposome which comprises an anti-disorder effective amount of the arachidonic acid metabolite. Generally, the anti-disorder effective amount of the metabolite is at least about 10⁻¹² g of the metabolite per kg of body weight of the animal, and is typically from about 10⁻¹²g per kg to about 10⁻³ g per kg. Desirably, the effective amount of the metabolite is from about 10⁻⁸ g per kg of body weight to about 10⁻⁴ g per kg. More desirably, the effective amount of the arachidonic acid metabolite is about 10⁻⁶ g per kg of body weight.

The liposome used in the method of this invention can comprise a drying protectant, and can de dehydrated, stored and then reconstituted prior to use. The drying protectant is preferably a saccharide such as maltose, lactose, sucrose, dextrose, raffinose or trehalose. Preferably, the saccharide drying protectant is maltose.

The method of this invention can comprise administering an additional bioactive agent, such as an anti-inflammatory or antimicrobial agent, to the animal.

### BRIEF DESCRIPTION OF THE DRAWINGS

FIGURE 1. Effect of LUV-PGE1 on Platelet Aggregation. LUV-PGE1 ("C-53": prepared in accordance with the procedures set forth in Example 1, below). X-axis: control, 0.1 microgram, 0.6 microgram, 1.1 microgram and 1.1 microgram post-administration LUV-PGE1 per kg of body weight (darkly shaded: collagen: lightly shaded: U46613). Y-axis: percent of control.
FIGURE 2. Heart Rate vs. Experiment Duration in treated/Control Dogs. Filled squares: LUV-PGE1: filled triangles: control: *: empty liposomes: filled diamonds: free PGE1.
FIGURE 3. Left Atrial Pressure vs. Experimental Duration. Filled squares: LUV-PGE1: filled triangles: control: *: empty liposomes: filled diamonds: free PGE1.
FIGURE 4. Infarct Size as Percent of Zone at Risk. X-axis: LUV-PGE1, free PGE1, empty liposomes, control. Y-axis: infarct size/zone at risk (%).
FIGURE 5. Myeloperoxidase Release From Myocardial Tissue. X-axis: infarct zone, border zone, risk region, control zone (darkly shaded: control: slanted lines: empty liposomes: straight lines: free PGE1: cross-hatched: LUV-PGE1.
FIGURE 6. Prevention of Lung Injury After Skin Thermal Injury With LUV-PGE1 Treatment. X-axis: control, burn, burn plus LUV-PGE1. Y-axis: percent albumin leak (cpm right lung/cpm blood).
FIGURE 7. Effect of Empty Liposomes or Free PGE1 on Lung Leak in Rats Given IL-1 Intratracheally. X-axis: saline control, IL-1, IL-1 + LUV-PGE1, IL-1 + empty liposomes, IL-1 + free PGE1. Y-axis: lung leak (cpm lung/cpm blood).
FIGURE 8. Rat Endotoxemia Model. X-axis: time (days) post-LPS administration: y-axis: percent survival in treatment group. Filled squares: rats administered saline control (0 µg/kg LPS): open squares: rats administered 10 µg/kg LPS; filled diamonds: 15 µg/kg LPS; open diamonds: 25 µg/kg LPS; filled triangles: 50 µg/kg LPS; open triangles: 75 µg/kg LPS; filled circles: 100 µg/kg LPS.
FIGURE 9. Inhibition of Secretion of Human Monocyte TNFα and IL-1β in Response to Lipopolysaccharide (LPS). X-axis: free PGE₁, LUV-PGE₁ (unilamellar liposomes containing PGE1 and prepared in accordance with the procedures described in this Example), placebo LUVs (large unilamellar liposomes not containing PGE1), placebo LUVs plus free PGE₁); y-axis: percent inhibition of TNFα and IL-1β secretion; unshaded: TNFα: shaded: IL-1β.
FIGURE 10. Attenuation of LPS-Induced Mortality. X-axis: time (days) post-LPS administration: y-axis: percent survival in treatment group. Filled squares: saline control (no LPS administered); filled diamonds: LUV-PGE₁; open diamonds: placebo LUVs plus free PGE₁; filled triangle: placebo LUVs; open triangles: LPS control (no liposomes or PGE₁); open squares: free PGE₁.
FIGURE 11. Elimination of Free PGE₁-Induced Mortality. X-axis: Saline control (no LPS, PGE₁ or liposomes). LPS control (LPS, but no liposomes or PGE₁). LUV-PGE₁, placebo LUVs plus free PGE₁, LATEX microspheres plus free PGE₁, placebo LUVs, LATEX microspheres: y-axis: percent survival in treatment group.

### DETAILED DESCRIPTION OF THE INVENTION

This invention provides a method for the manufacture of a pharmaceutical composition comprising formulating an arachidonic acid metabolite according to claims 1-23. The composition comprises a pharmaceutically acceptable carrier and the arachidonic acid metabolite in association with a unilamellar liposome. The composition is suitable for administration to an animal. Preferably, the animal is a human and the administration comprises intravenous administration.

"Pharmaceutically acceptable carrier" as used herein means any of the standard carriers, diluents, excipients and the like generally intended for use in connection with the administration of bioactive agents to animals, particularly humans. Such carriers are well known in the art and are generally chosen with regards to a number of factors, such as the particular drug being used and the intended route of administration, which are well understood by the ordinarily skilled artisan, or are within his purview to determine without undue experimentation. Suitable carriers include, but are not limited to salt solutions such as physiological saline (10 percent weight by volume sodium chloride in water), aqueous dextrose solutions, e.g., D5W (5 percent weight by volume dextrose in water), and the like, The pharmaceutical composition can further comprise auxiliary agents such as preservatives, anti-oxidants and the like in amounts, and for reasons, well known to the ordinarily skilled artisan.

Liposomes are self-assembling structures comprising one or more bilayers of amphipathic lipid molecules, each of which bilayers surrounds an aqueous compartment. Accordingly, liposomes can be unilamellar. i.e., have a single lipid bilayer, or liposomes can be multilamellar, i.e., have two or more lipid bilayers. The liposome of this invention is a unilamellar liposome. Preferably, the liposome is a large unilamellar liposome (LUV), and more preferably, is an LUV with a diameter of about 100 nm.

Liposomes can be prepared by a variety of techniques well known to ordinarily skilled artisans. For example, see Deamer and Uster, "Liposome Preparation: Methods and Mechanisms," in: Liposomes (M. Ostro, ed.), Marcel Dekker (New York), pp. 27-51 (1983); Cullis et al., in: Liposomes, From Biophysics to Therapeutics (M. J. Ostro, ed.), Marcel Dekker, pp. 39-72 (1987)). Bangham's procedure (J. Mol. Biol. 13:238 (1965)) produces "ordinary" multilamellar vesicles (MLVs). i.e., liposomes with two or more lipid bilayers, and involves dissolving one or more amphiphilic lipids in one or more organic solvents. The lipids are then dried, and the dried lipids are rehydrated with an aqueous solution so as to form the MLVs. Lenk et al. (U.S. Patent Nos. 4,522,803, 5,030,453 and 5,169,637), Fountain et al. (U.S. Patent No. 4,588,578) and Cullis et al. (U.S. Patent No, 4,975,282) disclose methods for producing multilamellar liposomes having a solute entrapped in their aqueous compartments, the concentration of the solute in each of the compartments being substantially equal. Unilamellar liposomes can be formed by ether or ethanol injection or infusion methods. Unilamellar liposomes can be produced from multilamellar liposomes by extrusion of the multilamellar liposomes, under pressure, through filters with defined pore sizes according to the disclosures of Cullis et al. (U.S. Patent No. 4,975,282) and Loughrey et al. (U.S. Patent No. 5,059,421). Extrusion, as well as such procedures as homogenization, milling sonication and French Press application can be used to reduce liposome size, which can be determined by such procedures as free-fracture electron microscopic examination of the liposome, as well as quasi-electric light scattering.

"Arachidonic acid metabolites" are prostaglandins, or compounds which can be converted to prostaglandins, e.g., artificially or in the body of an animal. Prostaglandins are a group of twenty-carbon fatty acids containing a five-carbon ring, plus seven- and eight-carbon chains, that are made from arachidonic acid and other twenty-carbon fatty acids having at least three double bonds (e.g., the "essential" fatty acids 8,11,14-eicosatrienoic acid, 5,8,11,14-eicosatetraenoic acid or 5,8,11,14,17-eicosapentanoic acid; see, e.g., Goodman and Gilman's The Pharmacological Basis of Therapeutics, *supra*). Arachidonic acid is the most abundant of these twenty-carbon prostaglandin precursors in humans.

The twenty-carbon essential fatty acid prostaglandin precursors, intermediates formed during prostaglandin synthesis, e.g., prostanoic acid, and structural analogs which can be converted to these compounds, are "arachidonic acid metabolites." "Prostaglandin-related compounds," e.g., leukotrienes, thromboxanes, lipoxins and prostacyclins, include those compounds which are functionally related to prostaglandins and which can also be derived from the twenty carbon essential fatty acid prostaglandin precursors. Prostaglandins, prostaglandin-related compounds, and eicosanoids, as well as structural analogs which can be converted to such compounds, are also "arachidonic acid metabolites." Preferably, the arachidonic acid metabolite administered to animals in accordance with the practice of this invention is a prostaglandin, more preferably, an E or I series prostaglandin, and most preferably, prostaglandin E1.

"Association" of an arachidonic acid metabolite with a liposome generally means that the metabolite is entrapped in the liposome's aqueous compartment, or is associated with the inner or outer monolayer of the liposomes bilayer, for example by way of electrostatic interactions between the metabolite and the headgroups of the monolayer's component amphipathic lipids. A preferred method for forming the unilamellar liposomal formulations of this invention is the association of the metabolite with the lipid in ethanol similar to the technique of Batzri et al., Biochim. et Biophys. Acta., 298:1016 (1973) using a transmembrane concentration gradient as disclosed in Bally et al. (U.S. Patent No. 5,077,056). In this technique, lipid and prostaglandin are co-dissolved in an aqueous-miscible organic solvent such as ethanol, then added slowly to a first aqueous solution. Optionally, a preservative such as butylated hydroxytoluene (BHT) may be admixed with the lipid in the solution.

The resulting liposome dispersion may be size-reduced to a more homogenous population, for example, by extrusion through a filter, preferably of 100 nm pore size, the filter being of either the straight path or tortuous path type. A preferred filter for use in this process is an aluminum oxide porous film such as the Anopore™ filters made by Anotec Separations. Such a population of liposomes may be formed by the extrusion procedures of U.S. Patent No. 5,008,050. Such extrusion procedures, wherein the liposomes are passed through a filter under pressure, allow the formation of homogenous populations of liposomes with regard to size. When the liposomes are passed more than one time through the filter, the number of passes required will be determined by that necessary to achieve the desired liposome size.

The filter sizes used in the invention are chosen according to the desired size of the final liposome product. In the present invention, liposomes having an average diameter of less than about 200 nm are preferred. The individual liposome used in this invention is preferably less than about 500 nm in diameter. More preferably, the liposome has a diameter of about 100 nm, as liposomes of this size are generally known to pass through the capillary bed of the lung and are therefore able to pass through to other organs and tissues. Therefore, a filter having a pore size of about 100 nm is preferably chosen for use in the extrusion step. The size-reduced liposomes may also be sterile filtered, such as by passage through a 220 nm Millipak filter (Millipore, Inc., Bedford, MA).

The unilamellar liposome used in the method of this invention comprises the metabolite, a lipid and a release-inhibiting aqueous buffer. The lipid preferably increases the strength of metabolite-lipid interactions, and thereby inhibits release of the metabolite from the liposome. Such lipids are "release-inhibiting lipids." Lipid based factors which tend to increase the strength of prostaglandin-lipid interactions include, but are not limited to, those factors which tend to make lipid bilayers less permeable to water and other small molecules, e.g., those factors which tend to increase Van der Waals, dipole-dipole and other interactions between acyl chains and hence, make acyl chains pack more closely together in the bilayer. For example, the number of double bonds in the bilayer's acyl chains can affect the chains' arrangement with respect to each other in the bilayer. The lower the number of double bonds, the more closely acyl chains are likely to pack together, and hence, are more likely to present a barrier to a prostaglandin transiting the bilayer. Accordingly, preferred lipids have saturated acyl chains. The preferred lipid with saturated acyl chains is dipalmitoyl phosphatidylcholine (DPPC). However, other saturated chain lipids can also be used.

Aqueous buffers in liposomes can also inhibit or prevent release of an arachidonic acid metabolite associated with a liposome. Such aqueous buffer are "release-inhibiting aqueous buffers." Characteristics of preferred release-inhibiting buffers include, but are not limited to, the ability to establish electrostatic repulsions with the metabolites or to enhance metabolite-lipid interactions. Furthermore, buffers with a higher buffering capacity, and hence a greater ability to maintain the desired pH, will be better release-inhibiting buffers. Preferred release-inhibiting buffers are citric acid buffers, particularly those citric buffers having a pH of about 4.5.

The method of this invention can be used to administer an arachidonic acid metabolite to an animal afflicted with a disorder characterized by cell activation and adhesion, inflammation and/or toxemia, amongst other indications. The method comprises administering to the animal an amount of the liposome comprising an anti-disorder effective amount of the metabolite. Disorders treatable in accordance with the practice of this invention include, without limitation: reperfusion injury, systemic inflammatory response syndrome (SIRS), adult respiratory distress syndrome (ARDS), myocardial infarction, vasculitis, burn injury, post-traumatic shock, vaso-occlusive disorders, arthritic disorders, such as rheumatoid arthritis, gout and filary arthritis, and auto-immune disorders such as systemic lupus erythematosus, juvenile diabetes, multiple sclerosis and Hashimoto's thyroiditis.

Certain disorders are characterized by the abnormal activation of cells, e.g., platelets and neutrophils, in the blood, and by the subsequent adhesion of these cells to each other or to activated cells in the surrounding vascular endothelium. Such cell activation and adhesion disorders are a significant problem in a wide variety of medical pathologies. Endothelial cells, for example vascular, plural, pericardial or abdominal endothelial cells, can be activated by cytokines, e.g., interleukin-1 (IL-1), tumor necrosis factor-alpha (TNF-alpha) or bacterial endotoxins. In like manner, blood cells, particularly neutrophils and platelets, can be activated by agents such as GM-CSF, bacterial endotoxins, bacterial chemoattractants, TNF-alpha and the C5a component of complement.

Activated cells have adhesion sites on their surfaces by which they can adhere to each other. Activated and adhered cells can form clumps, which can clog small blood vessels such as those found in the lungs and heart, and thereby reduce blood flow to surrounding tissue. The activated cells can also adhere to activated vascular endothelial cells; such adhesion can lead to subsequent degranulation of vascular endothelium, or to the release of mediators of cell damage, such as superoxide anion (O₂⁻) and proteolytic enzymes.

Amongst the cell activation and adhesion disorders to which the present invention is directed are reperfusion disorders, such as those related to the reperfusion of occluded blood vessels, or incidental to surgery in which blood flow is temporarily stopped (see, e.g., Seewaldt-Becker et al., "Effect of Anti-Adhesive Antibodies on Reperfusion Injury," (Springer et al., eds.) in: Leukocyte Adhesion Molecules, Springer-Verlag, New York (1990) pp. 138-148; and "Adhesion in Disease and Therapy," (Springer et al., eds.), in: Leukocyte Adhesion Molecules, Springer-Verlag, New York (1990), pp. 85-156). When there is a blockage in a blood vessel, surrounding endothelial cells, as well as downstream ischemic tissue, can be damaged. There can even be further damage to nearby endothelial cells when the occlusion is cleared. Such damaged cells can in turn induce activation in neutrophils and platelets after restoration of blood flow to the affected areas.

The same cells which become activated and subsequently undergo intracellular adhesion can also have surface receptors for arachidonic acid metabolites. Without intending to be limited by theory, it is believed that treatment with arachidonic acid metabolites, by binding to these receptors, can reduce cell activation and adhesion disorder-associated damage by deactivating the cell surface receptors responsible for the elevated levels of intercellular adhesion.

PGE₁ and PGI₂ have been found (see, Jugdutt et al., "Dissimilacts of Prostacycn, Prostaglandin E Prostaglandin Myocardial Infarct ze after Coronarusion in Conscious," Circulation CH, 49(3):685-700 981) to have scant effect in reducing the infarct size in dogs which were reperfused after simulation of a myocardial infarction by placement of an occluder snare around a coronary artery. In the reported tests, the prostaglandin was administered via continuous arterial infusion over a six-hour period, resulting in the administration of a relatively large dose of the drug. The need for this continuous infusion is believed to be that free, i.e.. non-liposomal, prostaglandins such as PGE₁ have an extremely short half life in vivo, and have to be continuously replenished to maintain an effective blood level. Furthermore, it is believed that the prostaglandin is rapidly inactivated when the blood passes through the lungs, which necessitates arterial infusion rather than a simpler intravenous administration. Additionally, the distribution of high levels of PGE₁ in vivo is known to induce systemic effects such as hypotension, tachycardia and diarrhea.

When patients are subject to the insults that can lead to ARDS, such as, to trauma, burns, sepsis, aspiration and hyperoxia, many organs in the body other than the lungs can be affected. The causes and clinical courses of this condition can vary widely. For example, in the case of a patient with a severe infection endotoxin is released from the bacterial cell walls, the inflammatory cascade is initiated, leading to septic shock. Again, as sepsis/trauma syndrome is not limited in causation to infections it is possible that no endotoxin is involved, but nonetheless, the release of factors such as TNF, IL-1 complement and leukotrienes is triggered.

Angioplasty is a technique whereby a balloon is inserted into an occluded artery and inflated in order to open blocked blood vessels. Although this technique has become quite routine in the management of coronary artery disease in the six month period following this procedure, over 33% of the treated patients experience restenosis, or reocclusion of the previously opened blood vessel. It is thought that this condition starts with injury to the vascular endothelium which often results form the balloon procedure. The exposed extracellular matrix will rapidly bind to several layers of activated platelets. Once platelets bind, they will release a variety of growth factors which will result in the proliferation of smooth muscle cells underlying the vessel to the point where the vessel becomes reoccluded. By preventing platelets from binding to the extracellular matrix, one can disrupt the cascade of events resulting in restenosis. Thus, acute administration at the time of the angioplasty procedure of a drug that prevents platelet adhesion could prevent restenosis.

Recently, De Servi et al., European Heart Journal, "Prostaglandin E administration in unstable angina patients undergoing PTCA; preliminary results", August 1990., published the results of a clinical trial in patients with unstable angina who were given an intracoronary infusion of PGE₁ prior to and following angioplasty. The drug was infused over a 24-hour period. The results of this study showed that the rote of restenosis six months after angioplasty in the PGE₁-treated group was reduced by almost 50% verses the untreated control group, even though the treatment with PGE₁ only lasted 24 hours.

Acute myocardial infarction (more commonly referred to as a heart attack) refers to a blockage of the blood supply to the muscles of the heart, usually caused by a blood clot. If the blood is prevented from reaching the heart for too long, the patient will die, When an occlusion of the coronary artery occurs, the patient is either treated with a fibrinolytic agent, such as tissue plasminogen activator (tPA) or streptokinase, to dissolve the clot, or the blockage may resolve itself. In both instances, blood flow is resumed to the ischemic (oxygen-deprived) region of the heart, This reflow of blood into the heart is called reperfusion. While reperfusion is necessary to save the patients life, it causes further injury to the heart muscle called reperfusion injury, Reperfusion injury is known to be the end result of the inflammatory cascade.

In addition to the problem of reperfusion injury following clot removal, patients suffering from a myocardial infarction may suffer from other secondary problems. For example, after the normal blood flow is restored to the heart, both neutrophils and platelets are activated. Activated platelets often adhere to one another and begin to reocclude the coronary artery, resulting in a situation where the rate of blood flowing to the heart decreases over time. In some cases, complete reocclusion will occur. PGE₁, in addition to preventing neutrophil binding to endothelial cells, prevents platelet aggregation and reduces, if not eliminates, the no reflow phenomenon.

Sharma et al., The American Journal of Cardiology, "Intracoronary Prostaglandin E₁ Plus Streptokinase in Acute Myocardial Infarction" , page 1161, Dec. 1986, vol. 58, has shown in a clinical setting of acute myocardial infarction that administration of free PGE₁ by slow intracoronary infusion together with intracoronary streptokinase provides positive clinical results when compared with a control group taking intracoronary streptokinase alone. The results showed decreased time to reperfusion, reduced dose of streptokinase required, increased percentage of vessels patent after 10 days, and higher ejection fractions. Drawbacks of the study are that the drug must be given by slow intracoronary infusion which is cumbersome and requires specialized facilities and highly trained personnel. Also this approach requires careful titration of the dose of PGE₁ so that significant drops in blood pressure can be seen.

Inflammation is a process of cytological and histological reactions occurring in affected blood vessels, and surrounding tissues, in response to an injury (see, e.g., Stedman's Medical Dictionary (Illustrated) (24th edition, J. V. Basmajian et al., eds.), Williams and Wilkins, Baltimore, MD (1982), pp. 707-708). Inflammatory responses to such stimuli include local reactions and resulting morphological changes, destruction or removal of injurious materials and activation of repair mechanisms. Thus, inflammation can be part of the process by which animals heal themselves.

However, inflammation can also occur in response to abnormal physiological stimuli and can cause problems in the body. Joints, for example, become inflamed in arthritic conditions such as gout, filary arthritis, rheumatoid arthritis and Lyme disease (see, e.g., Stedman's Medical Dictionary (Illustrated), *supra* at pages 123-124). These states may be characterized by the extravasation of cells, i.e. the egress of cells from the circulation into the inflamed area. Agents, such as prostaglandins, which can inhibit such extravasation, or which can otherwise inhibit inflammatory responses to abnormal physiological stimuli, can be used to ameliorate the inflammation.

Toxemia is the clinical manifestations observed during the course of infections by infectious agents, e.g., microbes which contain toxins and other substances poisonous to host animals. For example, during infections by certain gram-negative bacteria such as E. coli, a lipopolysaccharide (LPS) is released from the cell wall as it is broken down. The LPS can then induce the death of cells in the host animal. Toxemic conditions occur in animals in which toxins such as LPS are made available, i.e., in septic conditions, or conditions of systemic disease caused by the multiplication of microorganisms in the circulation (see. e.g., Stedman's Medical Dictionary (Illustrated), *supra* at pages 1274-1275 and 1464). Toxemia can also result from exposure of the animal to traumatic stimuli, e.g.. physical or chemical trauma.

"Auto-immune disorders," such as systemic lupus erythematosus, juvenile diabetes, multiple sclerosis and Hashimotos thyroiditis, are characterized by an animals immune system attacking its own tissues.

"Anti-disorder effective" amounts of an arachidonic acid metabolite are any amounts effective to ameliorate, inhibit or prevent the cell activation and adhesion, inflammation, toxemia, or other indication associated with the disorder being treated according to the method of this invention. Typically, the effective amount of the metabolite comprises at least about 10⁻¹² g of the metabolite per kg of body weight of the animal, and desirably, from about 10⁻¹² g per kg to about 10⁻³ g/kg. More desirably, the effective amount of the metabolite comprises from about 10⁻⁸ g per kg of body weight to about 10⁻⁴ g per kg. Most desirably, the effective amount comprises about 10⁻⁶ g of the arachidonic acid metabolite per kg of body weight of the animal.

Generally, a significantly lower dosage of the liposomal metabolite, in comparison to a dosage of the free metabolite, is required to obtain the desired effect. As discussed above, because of the rapid metabolism of free prostaglandins in vivo, long continuous infusions of relatively large doses of these drugs have been required to maintain an effective blood level in the patient being treated. However, the hypotension, tachycardia and diarrhea caused by high blood levels of prostaglandins limit the amounts of free prostaglandins which can be administered. Furthermore, the high cost of prostaglandins makes it prohibitively expensive to administer such large dosages. The method of the present invention provides for the effective administration of the arachidonic acid metabolites at reduced cost and with reduced side effects.

Arachidonic acid metabolite. e.g., prostaglandin, treatment can reduce the damage exhibited in those animals afflicted with disorders characterized by cell activation and adhesion, toxemia, inflammation and other indications. The same cells which have receptors for cellular activating agents can also have surface receptors for the metabolites. It is believed that when metabolites bind to their receptors, they can deactivate the surface receptors responsible for the elevated levels of intercellular adhesion. The mechanism for this deactivation is believed to be a protein kinase A-mediated increase in intracellular cAMP levels instigated by metabolite/receptor interaction. Without cell-cell binding, cofactors such as O₂⁻ and various degradative enzymes cannot be released, and tissue damage is eliminated.

PGE₁ has been shown to be a potent inhibitor of both neutrophil and platelet aggregation, as well as the binding of these cells to activated vascular endothelial cells.. Arachidonic acid metabolites such as PGE₁ are also believed to have the ability to both prevent inflammation, and to turn it off once it has been initiated. It has been found that the extracellular release by neutrophils of mediators of inflammation can be modulated by the elevation or depletion of intracellular stores of cyclic adenosine monophosphate (cAMP).

It has been found that the extracellular release by neutrophils of mediators of inflammation can be modulated by the elevation or depletion of intracellular stores of cyclic adenosine monophosphate (cAMP) and cyclic guanosine monophosphate (cGMP). Elevation of cAMP reduces release of mediators of inflammation whereas increases in the levels of cGMP enhances the excretion of those mediators. cAMP is sometimes referred to as the "universal off-switch" since increasing intracellular levels of cAMP can turn off inflammation, regardless of the factor that initially turned it on. Importantly, some prostaglandins such as PGE₁ elevate cAMP, thus providing the rationale for using PGE₁ as an anti-inflammatory agent. PGE₁ can be thought of as an agent that can activate the "universal switch off". It has now been shown in vitro that PGE₁ inhibits the binding of neutrophils and platelets to themselves, as well as to endothelial cells, by preventing the activation of the receptors necessary to mediate cell-cell adhesion. This inhibition is coincident with the elevation of intracellular cAMP. Without cell-cell binding, cofactors such as O2- and various degradative enzymes cannot be released, and tissue damage is eliminated. Therefore, PGE₁ actually acts as a potent anti-inflammatory agent. It can both prevent inflammation and turn it off, regardless of whether the mediating factor is IL-1, TNF, complement, leukotrienes or others.

Liposomes can be particularly advantageous vehicles for delivering prostaglandins to their intended sites of action. Without being bound by a particular theory or mechanism, it is believed that the liposomes are attracted to the activated cells and adhere to the activated surfaces. The prostaglandin is then readily available at the site of injury to deliver its anticellular adhesion action. One theory for the attraction of liposomes to adhesion activated cells is that liposomes are opsonized by fibronectin and vitronectin in the blood. Opsonization is the process by which bacteria are altered such that they become more readily and efficiently engulfed by phagocytes. Thus opsonized liposomes would be more readily attracted to the activated neutrophils which express receptors for fibronectin and vitronectin, thereby delivering the associated prostaglandin to the affected sites.

Liposomal arachidonic acid metabolite formulations in which the metabolite is associated with the liposome by way of a pH gradient across the liposome's lipid bilayer can be therapeutically useful. Liposomal formulations having an internal acidic aqueous buffer, such as a citric acid buffer, particularly a pH 4.5 citric acid buffer, are preferred for establishing transbilayer pH gradients. Arachidonic acid metabolites associated with liposomes by such gradients tend to remain associated with the liposome as long as the pH gradient is maintained. However, when the gradient decays in the bodies of animals to which the liposome has been administered, and the internal pH consequently increases, arachidonic acid metabolites generally become unassociated with the liposome. The metabolite then is more likely, than when it is associated with a liposome, to be able to interact with the corresponding surface receptors on cells, such as neutrophils, that become activated and that subsequently undergo intercellular adhesion.

Unilamellar liposomes used in accordance with the practice of this invention can comprise a drying protectant, which is generally a hydrophilic compound, such as a saccharide, urea, dextran, albumin or polyvinyl alcohol, capable of preventing the rearrangement of the lipids in the liposomes, so that when the liposomes are reconstituted subsequent to dehydration, a substantial portion of the contents originally entrapped in the liposomes remain therein. Dying protectants are generally strong hydrogen bond acceptors, and typically possess stereochemical features favorable to preserving the intramolecular spacing of the bilayer constituents. Saccharides, such as mannose, galactose, trehalose, raffinose, maltose, sucrose, lactose or dextrose are preferred drying protectants. Maltose is particularly preferred.

Saccharides are typically used as drying protectants at concentration of from about 5 to about 20 percent, preferably at about 10 percent by weight of the aqueous phase used to prepare liposomes. Mannitol may be used in conjunction with any of the saccharides, but it has surprisingly been found that when used alone, mannitol does not succeed in maintaining liposome size. Mannitol may be used in concert with the saccharides in about a 0-2% concentration, preferably a 1% concentration. The total concentration of saccharide used ranges from about 5% to about 20%, preferably 10% to 12%, most preferably about 10%, Additional preservatives such as BHT or EDTA in the formulations at, for example, 5 mg BHT per ml of ethanol, and, for example, 0.01% EDTA in 10% dextrose may also be included.

Liposomal dehydration enables liposomes to be stored for extended periods of time; they can then be reconstituted on an as-needed basis for administration to subjects. Dehydration is preferably carried out using a dying protectant in connection with the liposomes, in accordance with the procedures of Schneider et al. (U.S. Patent No. 4,229,360) and Janoff et al., (U.S. Patent No. 4,880,635); the contents of which are incorporated herein by reference). Alternatively, the dying protectant can be omitted if the dehydration is conducted without prior freezing and sufficient water is left remaining in the liposomal preparation to maintain the integrity of a substantial portion of the liposomal bilayers through the dehydration-rehydration process.

Lyophilized prostaglandin-liposome formulations can be stable for at least one year when stored at 6o C or 25 deg. C. Insertion of the arachidonic acid metabolite into the liposome membrane, followed by lyophilization, is believed to shield the metabolite from water, and to thereby inhibit hydrolysis. Stability studies using high pressure liquid chromatography (HPLC) analysis of the formulation have shown that after storage at 6°C for one year, no degradation products of PGE₁ were present. When the lyophilized liposomes are to be used, rehydration is accomplished by adding an aqueous solution, e.g., distilled water, water for injection (WFI), or buffer or aqueous solution of appropriate pH, as described above, to the liposomes. The liposomes can be then resuspended into the aqueous solution by gentle mixing. The rehydration can be performed at about 25 deg. C.

The method of this invention can comprise administering an additional bioactive agent to the animal, i.e., a bioactive agent in addition to the arachidonic acid metabolite with which the liposome is associated. "Bioactive agent" as used herein denotes any compound or composition of matter which can be administered to animals. These include agents having biological activity in the animals, as well as those useful for imaging or other forms of diagnosis. Bioactive agents include, but are not limited to: antiviral, antibacterial, antifungal, antiparasitic, antimetabolic, antiglaucomic, anti-inflammatory or antineoplastic compounds, sterols, carbohydrates, amino acids, peptides, proteins, immunoglobulins, immunomodulators, dyes, toxins, enzymes, hormones, neurotransmitters, glycoproteins, radiolabels, radiopaque compounds, fluorescent compounds, cell receptor proteins, cell receptor ligands, mydriatic compounds, bronchodilators, local anesthetics, growth promoting agents, regenerative agents and the like. Additional bioactive agents are selected for particular indications according to criteria, for example, the need to treat other conditions afflicting the animal, well known to ordinarily skilled artisans given the teachings of this invention. The additional bioactive agent can be an additional arachidonic acid metabolite.

This invention is more fully described in the following Examples. However, those of ordinary skill in the art will readily understand that these examples are merely illustrative of the invention as described in the claims which follow thereafter.

### EXAMPLES

### Example 1

### Liposome Preparation

A 1500 ml botch of liposomal PGE₁ was mode up of the following components:

| Ingredient | Per 1500 ml | Per ml |
|---|---|---|
| Egg phosphatidylcholine (EPC) | 7.06 g | 4.4 mg |
| Maltose monohydrate | 150.0 g | 100 mg |
| Ethanol (anhydrous) | 8.38 ml | 0.00558 ml |
| Butylated hydroxytoluene (BHT) | 45 mg | 0.03 mg |
| PGE₁ 15mg | 0.01mg | |
| Water for Injection, USP | qs 1500 ml | |

### Preparation:

1350 ml of water for injection was added to a beaker and set with a nitrogen sparge for at least 30 minutes. The 150 g of maltose (J.T. Baker, Phillipsburg NJ) was added to the water and mixed until dissolved, with the nitrogen sparge continued, This produced 1440 ml of mixture at a pH of 4.81.

In another beaker, the 7.06 g of egg phosphatidylcholine (EPC) (Nippon Oil and Fats, Hyogo, Japan) were combined with 6 ml of ethanol (anhydrous) and mixed until dissolved, and the 45 mg of BHT was added and mixed until dissolved. To this mixture, the 15 mg of PGE₁ was added and mixed until dissolved, the remaining 2.37 ml of ethanol being used to rinse any remaining PGE₁ in the weighing container into the mixture.

The ethanol solution was drown into a 10 ml capacity glass syringe and injected through a 14 gauge cannula slowly over a period of 11 minutes into the maltose solution with rapid mixing and continued nitrogen sparge. Upon addition of the ethanol/lipid mixture, the solution become cloudy, indicative of the formation of liposomes. The suspension was then diluted to a final volume of 1500 ml with the remaining water for injection.

The liposome dispersion was then extruded 3 times through a 0.2 um pore size Nucleopore® polycarbonate straight through path type filter (Nuclepore, Pleasanton CA), followed by 5 extrusions through a corresponding 0.1 um filter The particle size of the resulting liposomes was determined to be 0.169 um (S.D. 0.041 um), using quasi-elastic light scattering (QELS) (Nicomp Particle Sizer). The sized liposome dispersion was then passed through a 0.22 um Millipak sterilization filter. Finally, 10.5 ml aliquots of the dispersion were filled into vials and lyophilized according to the procedures set forth as Example 2 to form a lyophilized product, which can be used immediately or stored for future use.

The lyophilized product was rehydrated with 10 ml of 0.01 M acetate buffer (pH 4.3), with the resultant suspension having pH of about 4.3. Entrapment of the PGE₁ in the liposomes was determined by HPLC, and it was found that at least 98 percent of the available PGE₁ was entrapped in the liposomes, at a concentration of about 9.0 micrograms of PGE₁ per ml of rehydrated suspension.

### Lyophilization process

Split-top, butyl-rubber stoppered, Flint lyophilization vials of 50 ml capacity were filled with 10.5 ml of aqueous-suspended liposomes, containing PGE₁. These vials were placed on shelves in the lyophilizer (PV-24 Stokes Lyophilizer). The vials were held at 0°C for 1.5 hours. The shelf temperature was then decreased to -45°C at a rate of 0.8°C per minute, and held for 1 hour, after which a vacuum of 100 um Hg was applied. The shelf temperature was then increased to -28°C at a rate of 0.5°C per minute, while continuing the vacuum at 100 micrometer Hg.

The vials were held at -28°C for 50 hours at 100 um Hg vacuum. The shelf temperature was then increased to +25°C at a rate of 0.5°C/min and held for 22 hours. The vials were then stoppered under partial vacuum.

### Acute Myocardial Infarction/In vivo test of liposomal PGE₁

The rehydrated liposomes of Example 1 were tested in vivo as a means of reducing reperfusion injury incidental to the treatment of myocardial infarction resulting from occluded blood vessels. A total of 53 conditioned 25-35 kg dogs were studied in the dose-ranging trials and the infarction study. Dogs were excluded from analysis for the following reasons: extensive collateralization to the infarct zone (4), heart worms at necropsy (1) and cardiac arrest during occlusion (1). Forty dogs survived of which 27 were suitable for analysis: 7 dogs each in the control, liposomal PGE₁ (LUV-PGE₁) and liposome-control (empty, or "plain", liposomes) groups and 6 dogs in the PGE₁-control (free PGE₁) group.

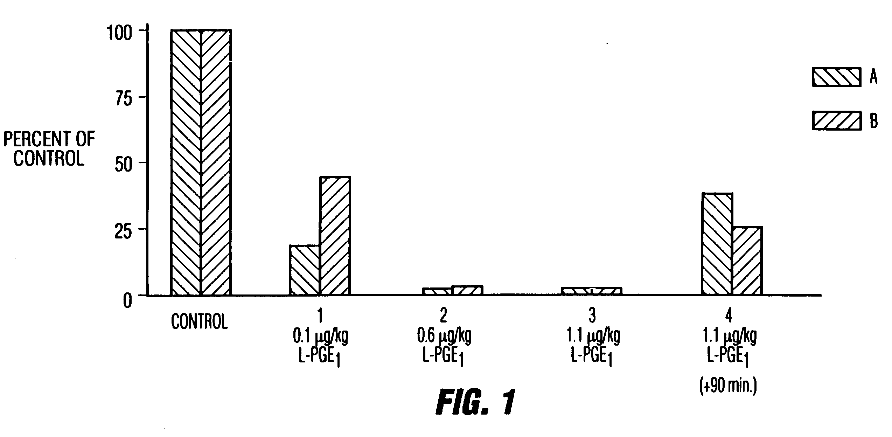

First, dose-ranging trials were conducted to determine a suitable dosage for use in the myocardial infarction studies. Incremental doses of liposomal PGE₁ were given while heart rate, mean arterial pressure, cardiac output and pulmonary capillary wedge pressure were measured. Blood samples were also taken at intervals for platelet aggregation studies (Figure 1). Doses greater than 2.0 µg/kg delivered as a bolus over 10 to 20 minutes produced a marked tachycardia and transient hypotension with a significant increase (doubling) in cardiac output. Platelet aggregation was partially inhibited at 0.1 µg/kg and totally inhibited at 0.6 µg/kg. A dose of 0.5 µg/kg delivered over 10 minutes seemed to increase the heart rate and cardiac output only slightly. In order to optimize the potential results, it was elected to give two doses of liposomal PGE₁ of 0.5 µg/kg each. One injection was administered just after occlusion of the infarct-related artery and the other just prior to reperfusion. This regimen should insure an effect throughout the period of ischemia and initial period of reperfusion. The effect of the liposomal PGE₁ appeared to persist for some time after administration. This observation is significant to reocclusion after reperfusion and restenosis following angioplasty which are directly related to platelet aggregation and adherence to endothelial cells and extracellular matrix.

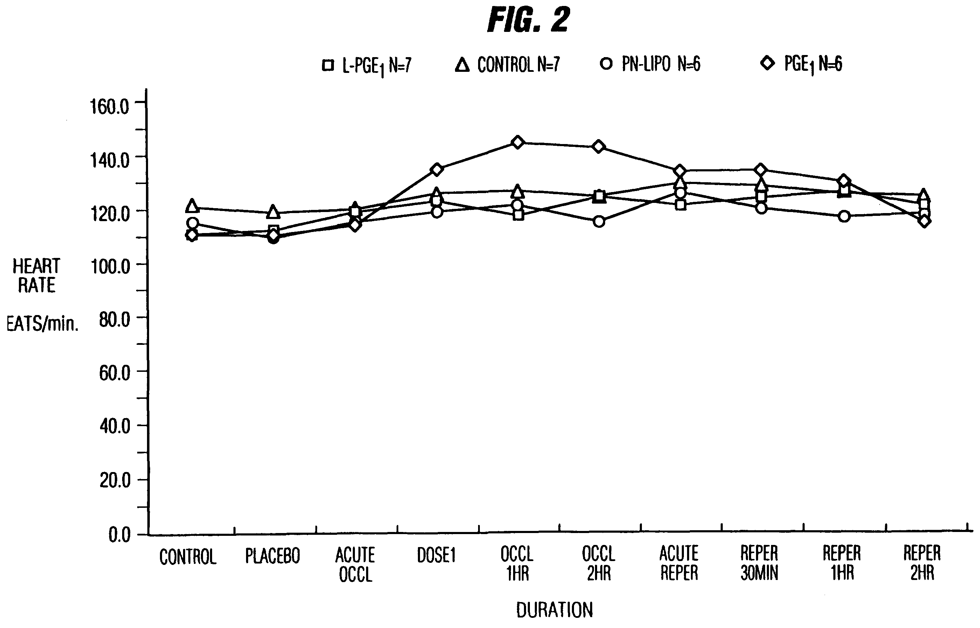

The effects of liposomal PGE₁, free PGE₁, empty liposomes and a control group on heart rate were studied. The test dogs were anesthetized with sodium pentobarbital and maintained with intravenous pentobarbital and Inovar® (droperidol/fentanyl). Arterial occlusion was simulated by ligating the left descending aorta artery. Ten minutes after occlusion, 0.5 µg/kg liposome-bound PGE₁ was administered intravenously over ten minutes into a first group of dogs, with a second group of control animals maintained without treatment. The liposome infusion tended to cause an increase in heart rate which was counteracted, in part, by administration of small amounts of additional Inovar. Tests showed no major differences in heart rate throughout the experiment between the control and treated animals. One hundred minutes after occlusion, a second dose of 0.5 µg/kg was administered over ten minutes. After 2 hours, the ligature was removed, causing an acute reperfusion of the blood vessel. Two hours later, the dog was euthanized, and the heart examined for myocardial infarction damage. The results are summarized in Figure 2. The group receiving free PGE₁ exhibited a significant rise in heart rate after the initial dosage and continuing until reperfusion. This rise was not noted in any of the remaining 3 groups of animals. This rise has been attributed to compensatory tachycardia due to vasodilation.

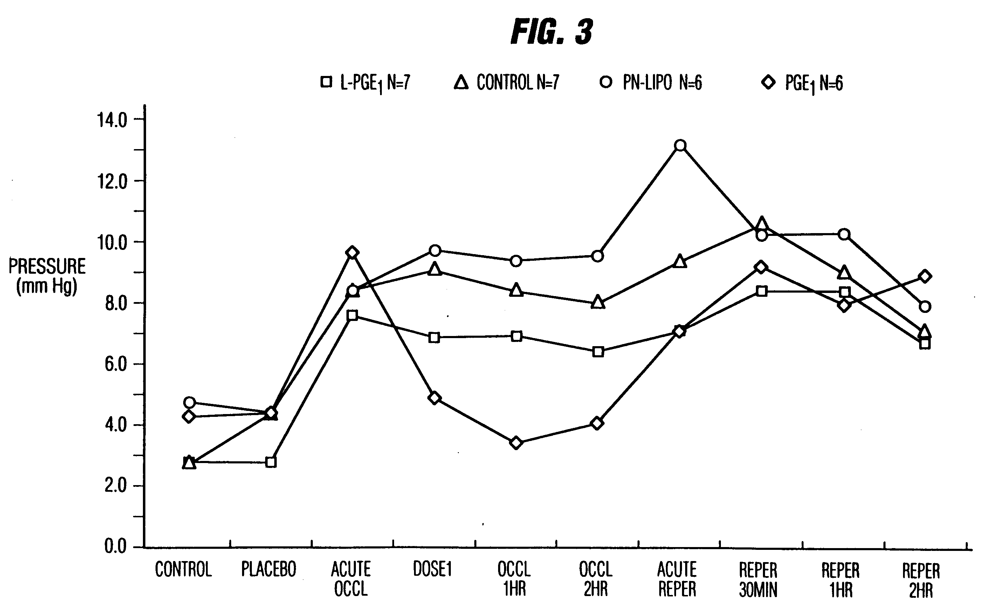

A total of seven dogs were included in the control group, and seven dogs were treated with liposomal PGE₁. During the test period, mean aortic pressure remained relatively constant, and there was no significant difference between the control and treated groups. Mean left arterial pressure increased in each group after occlusion, but there were no significant differences between the groups. Results are summarized in Figure 3.

As anticipated, myocardial blood flow, as measured by 15 um radioactive microspheres, demonstrated a significant decrease in all groups of animals after occlusion. There was no significant improvement in collateral blood flow after liposomal PGE₁ infusion in the test animals suggesting that the microvascular vasodilatory effects of liposomal PGE₁ were minimal.

With reperfusion blood flow increased significantly higher in treated dogs compared to control animals. Examination of the hearts showed that the myocardial infarct size, expressed as a percent of region at risk (i.e. area of low flow during ischemia), appeared reduced by about 50% in the dogs treated with liposomal PGE₁, as compared to the untreated control dogs. In addition, the administration of liposomal PGE₁ resulted in an almost total inhibition of platelet aggregation in the blood.

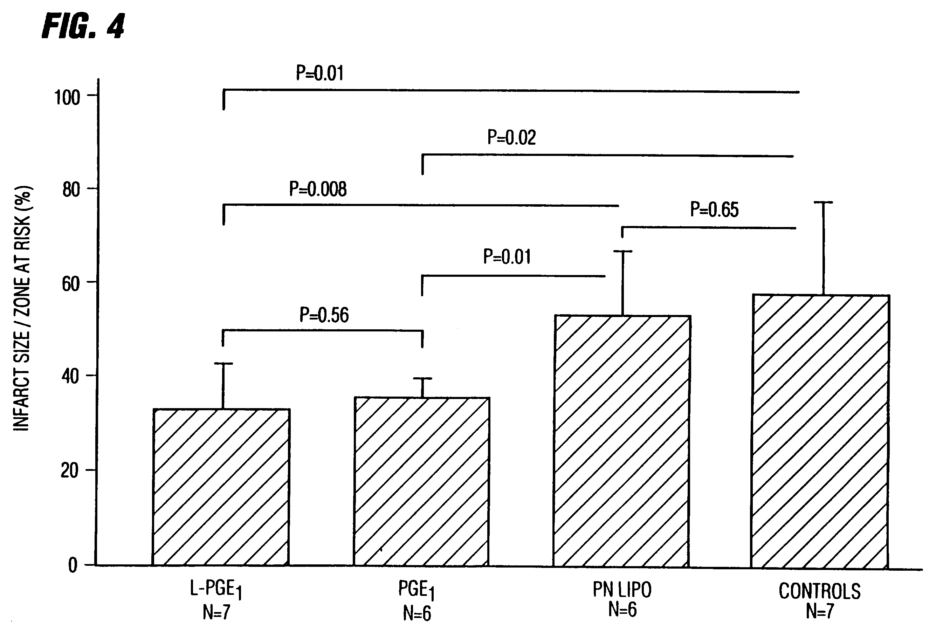

Figure 4 summarizes the results on the four groups of dogs receiving either liposomal PGE₁, free PGE₁, empty liposomes or saline control. The liposomal PGE₁ was given in two 0.5 µg/kg infusions over 10 minutes each for a total dosage of 1.0 µg/kg. Free PGE₁ was continuously infused over 90 minutes at .1 µg/kg/minute or at a total dosage of 9 µg/kg.

Tests were also conducted to measure the white blood cell infiltration into the ischemic tissue of the heart. Immediately following extraction of the heart from the animals, samples were obtained from the 1) infarct zone, 2) border zone of the infarct zone within the risk region, 3) risk (ischemic) zone, and 4) control zone outside the region at risk. (The region at risk was the portion of the heart supplied by the occluded blood vessel.) The tissue was flash frozen using liquid nitrogen, and kept at -70°C until analyzed. Myeloperoxidase (an enzyme found only in neutrophils) activity was subsequently analyzed for each of the four zones in each animal heart. Initially, six control animals were tested in this manner, although one of these (CONT 4 in the table) was considered aberrant throughout the test, but was included in the tabulation for completeness. Only four of the animals treated with liposomal PGE₁ (LIPO 1-4) were included in this portion of the study. The results are presented in tabular form in Table 1, with the level of myeloperoxidase expressed in arbitrary units of enzyme for comparative purposes:

**TABLE 1**

| MYELOPEROXIDASE RELEASED FROM MYOCARDIAL TISSUES | | | | |
|---|---|---|---|---|
| | ZONE | | | |
| DOG | 1 | 2 | 3 | 4 |
| CONT 1 | 22.8 | 17.8 | 1.1 | 1.3 |
| CONT 2 | 18.2 | 0 | 6 | 0 |
| CONT 3 | 23.3 | 29.0 | 17.9 | 0.7 |
| CONT 4 | 0 | 0 | 0 | 0 |
| CONT 5 | 23.5 | 24.4 | 19.6 | 3.9 |
| CONT 6 | 2.7 | 3.0 | 1.2 | 1.7 |
| MEAN | 15.1 | 12.4 | 7.6 | 1.3 |
| STD | 10.9 | 13.0 | 8.9 | 1.5 |
| LIPO 1 | 17 | 0 | 0 | 0.6 |
| LIPO 2 | 1.3 | - | 0 | 0.5 |
| LIPO 3 | - | 0 | 0 | 0 |
| LIPO 4 | 2.7 | 0.5 | 0 | 0.2 |
| MEAN | 1.9 | 0.2 | 0 | 0.3 |
| STD | 0.7 | 0.3 | 0 | 0.3 |

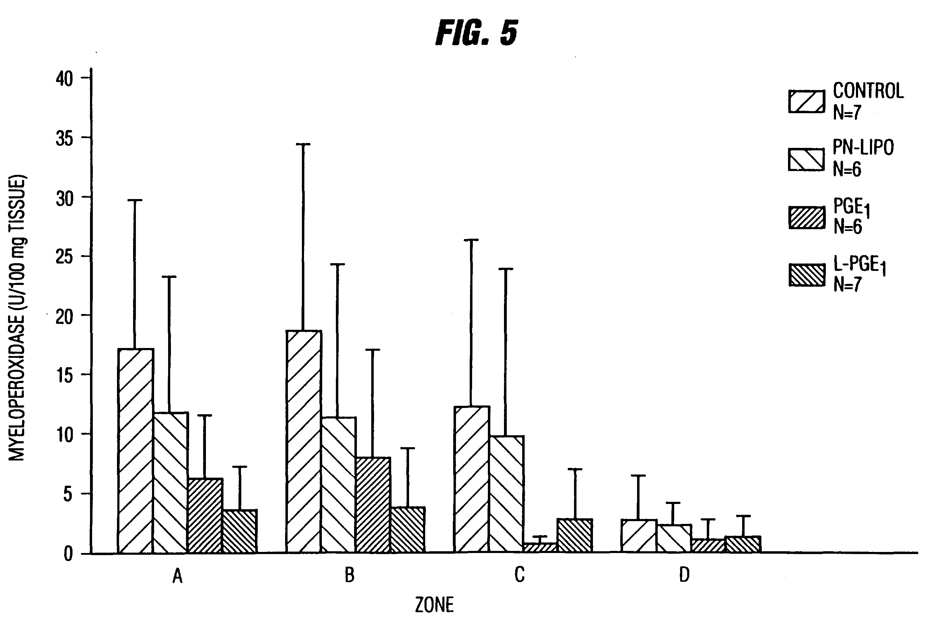

Additional dogs were tested in each group. Figure 5 summarizes the results of the totality of dogs suitable for analysis in each of the four groups tested.

In the above table, a dash indicates no measurement was made for that particular value.

### EXAMPLE 3

### In vivo tests for prophylaxis of ARDS:

Adult respiratory distress syndrome (ARDS) is a condition secondary to a variety of insults including, but not limited to, trauma, burns aspiration and hyperoxia. The condition is characterized by interstitial edema die to capillary injury. Once the lungs fill with fluid, breathing becomes difficult, and 5-60% of the patients die. The sequence of events leading to this catastrophic conclusion can vary depending upon the nature of the initial insult. Factors such as C3a, C5a, IL-1 and TNF activate the neutrophils. The mode of action of PGE₁ is independent of the mechanics of cellular activation. In the presence of PGE₁, neutrophils cannot be activated, and if they are already activated they will be turned off, thereby preventing the disease.

Studies were conducted on rodent models for ARDS where lung injury had been induced by either thermal injury or by direct intratracheal injection of IL-1. As noted in the following examples, doses of liposomal-PGE₁ prevented the leak of fluid into the lung and significantly reduced the influx of neutrophils into the lung.

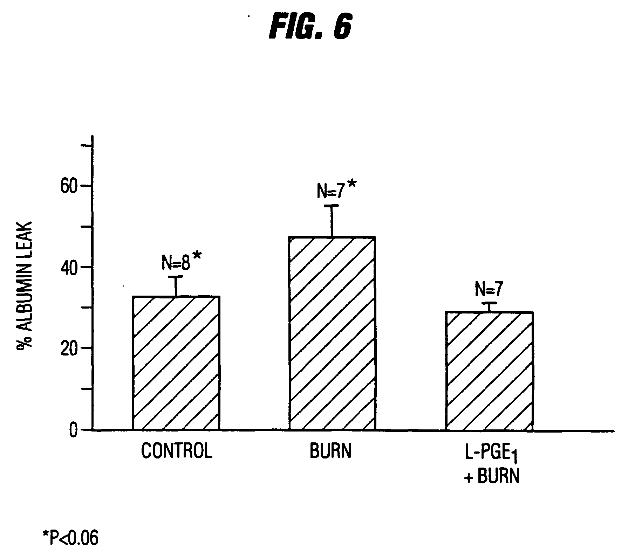

The rehydrated liposomal PGE₁ of Example 1 was tested in vivo as a prophylactic to the onset of adult respiratory distress syndrome (ARDS). Test rats were divided into three groups containing seven or eight animals each and treated with either 8 µg/kg of liposomal PGE₁ or saline simultaneous with and one after thermal injury (by immersion in 70°C water for 45 seconds). The first group was treated with liposomal PGE₁. the second group was not treated with the liposomal PGE₁, but was subjected to the same trauma and the third group was neither treated nor traumatized One hour prior to sacrifice of the rats, 125 l-albumin was injected intravenously as a marker for fluid leak into the lung. Four hours after thermal injury the animals were sacrificed.. The results of the study are summarized in Figure 6.

An effect of such traumatization is known to be the onset of ARDS, as evidenced by an increased level of albumin in the lungs of affected animals. The untreated traumatized animals all exhibited elevated levels of albumin in the lungs. Furthermore, six out of these seven test animals died. The level of albumin in the animals treated with liposomal PGE₁ was found to be about as low, or even lower, than the levels in the untraumatized control group. Furthermore, none of the seven PGE₁ treated animals died as a result of the trauma. These results show the effectiveness of liposomal PGE₁ as a prophylactic to the onset of ARDS.

### In vivo tests for treatment of ARDS induced by intratracheal injection of IL-1 :

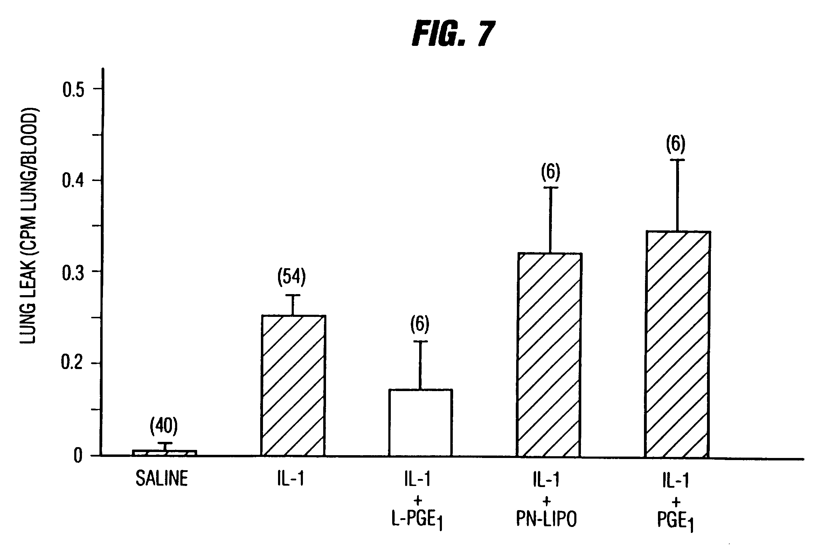

Intratracheal instillation of recombinant IL-1 induces a neutrophil influx and lung injury in rats (Figure 7). Liposomal PGE₁ treatment will decrease IL-1 induced lung injury and neutrophil influx. 50 ng of commercially available interleukin 1 (IL-1) or saline (in the control group) was instilled into the trachea of rats inducing neutrophil infiltration into the lungs and leak of fluid into the alveoli. Intravenous treatment, 6 µg/kg, of either rehydrated liposomal PGE₁ of Example 1, free PGE₁ or empty liposomes was administered 2.5 hours after IL-1 instillation in rats having received the IL-1. Of the rats analyzed 40 received only saline, 54 received IL-1 and were untreated, 6 received IL-1 and were treated with liposomal PGE₁, 6 received IL-1 and were treated with empty liposomes, and 6 received IL-1 and were treated with free PGE₁.

Four and a half hours after the IL-1 was given, after many neutrophils had already infiltrated into the tissue, the rats were injected with 125 l albumin as a marker into the bloodstream. A half an hour after the albumin was injected the animals were sacrificed, the lung removed and the leak of fluid into the lung was quantitated by determining the amount of isotope in the tissue. A lung leak index was calculated based on the count per minute (cpm) of labeled 125 l-albumin in the lung divided by the amount of labeled 125 l-albumin in the bloodstream. The lung leak is expressed on the Y-axis of Figure 7. As noted in Figure 7, instillation of IL-1 without treatment caused a 2.5-fold increase of lung leak above sham or animals given saline. However, when liposomal PGE₁ treatment was given after instillation of IL-1, the lung leak index was equal to that seen in the sham control. Importantly, treatment with neither the free PGE₁ nor the empty liposomes had any effect on lung leak.

This sequence of events represents that which might occur in a clinical setting. Injury was induced, and treatment was initiated at a later time after the appropriate diagnosis was made.

### Example 4

### Rat Endotoxemia Studies

### Preparation of EPC-Containing PGE₁ MLVs

An egg phosphatidylcholine (EPC) stock solution (20 mg/ml in ethanol) was prepared as follows: 1 g of dried EPC was dissolved in 50 ml of absolute ethanol, with gentle swirling, in a 50-ml brown bottle with a Teflon-lined lid. The resulting solution was stored at minus 20 degrees Celsius. A PGE₁ stock solution (1 mg/ml in ethanol) was prepared as follows: 20 mg of dried PGE₁ was transferred to a 20-ml vial, to which 20 ml of absolute ethanol was added. The PGE₁ was dissolved in the ethanol with gentle swirling; the resulting solution was stored at minus 20 degrees Celsius.

An aliquot of the EPC stock solution (9.75 ml), and an aliquot of the PGE₁ stock solution (0.5 ml), were combined in a 500-ml round-bottom flask; the ethanol was removed by rotoevaporation at about 30 degrees C. for at least two hours. The dried EPC/PGE₁ was resuspended in a pH 4.5 buffer (e.g., 50 mM acetate, 150 mM NaCl, pH brought to 4.5 with 10N NaOH; glass beads aided in resuspension of the dried EPC/PGE₁) so as to form a liposome suspension. This suspension was stored at 4 degrees C.

### Preparation of DPPC-Containing PGE₁ MLVs

A DPPC stock solution was prepared as described above, using 1.035 g of dipalmitoyl phosphatidylcholine (DPPC) dissolved in methylene chloride. Rehydration of the dried DPPC/PGE₁ mixture required heating in a water bath, with swirling, at about 52 degrees C. for about 3-5 minutes.

### Rat Endotoxemia Model

Fever, hypotension, changes in leukocyte counts and diarrhea are symptoms of gram-negative bacterial infections. These infections may lead to disseminated intravascular coagulation and irreversible shock. A large volume of literature indicates the involvement of leukocyte derived IL-1, IL-6 and TNFα in mediating the progression of endotoxic shock. Because our *in vitro* data indicated an inhibition of these cytokines from cultured monocytes, we developed an *in vivo* model of rat endotoxemia, using mortality as an end point, to assess the effectiveness of PGE₁ and particulate formulations in attenuating LPS-induced death.

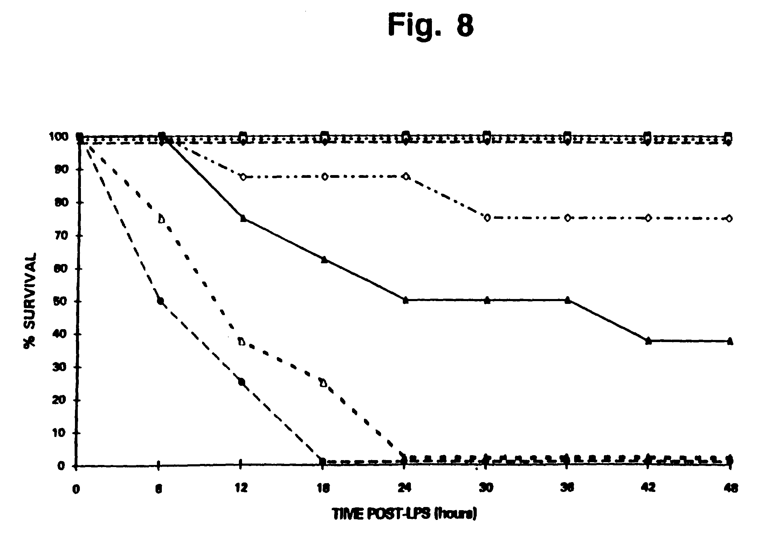

Experiments were designed to establish an LD₅₀ for E. coli LPS (lipopolysaccharide; serotype 055:B5) in Sprague-Dawley rats. The data from these experiments are shown in Figure 8, and indicate that the LD₅₀ is at 50 µg/kg. This LPS dosage was used in subsequent experiments, unless otherwise indicated.

Male Sprague-Dawley rats, weighing 126-150 g each, were acclimated for two days in an animal facility with food and water *ad libitum*. At time 0, groups of rats (n=16) were injected iv. with either E. coli lipopolysaccharide as a single bolus, or with a saline (no LPS) control. Mortality was assessed at various times (days) post-LPS administration.

### Inhibition of Tumor Necrosis Factor Alpha (TNFα and Interleukin-1 Beta (IL-1β) Synthesis in Response to Lipopolysaccharide (LPS) and PGE₁

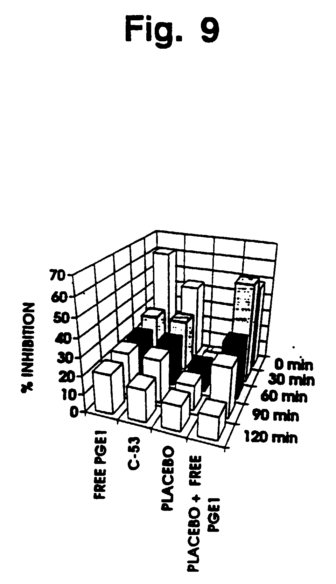

Adherent human monocytes were stimulated with LPS (1µ g/ml/10⁶ cells) at time 0. Free PGE₁ (not entrapped in liposomes), LUV-PGE₁ (large unilamellar liposomes (LUVs) prepared in accordance with the procedures described in this Example), LUV-PGE₁ "placebo" liposomes (LUVs not containing PGE₁). LUV placebo liposomes plus free PGE₁, placebo liposomes or a saline control (no PGE₁) were injected simultaneously (10 µM PGE₁). Secreted TNFα and IL-1β were assayed at three hours. Results from these experiments are presented in Figure 9.

### Attenuation of LPS-Induced Mortality

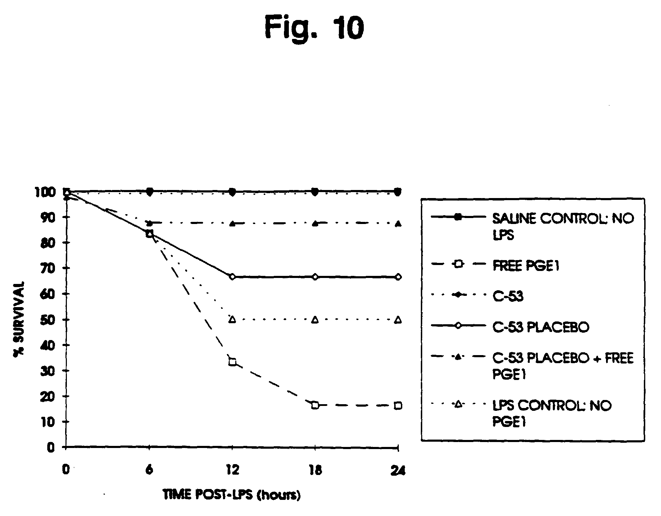

Male Sprague-Dawley rats were injected i.v. with 50 µg LPS/kg of body weight at time 0. Free PGE₁, LUV-PGE₁ (40 µg/kg PGE₁), placebo LUVs (LUVs not containing PGE₁; equivalent particle number to the number of liposomes given with the 40 µg/kg PGE₁--LUV-PGE₁ dose) or placebo LUVs (40 µg/kg lipid equivalency) plus free PGE₁ (40 µg/kg). There were 12 rats were in each treatment group. Survival was assessed in each group at 6, 12, 18 and 24 days post-LPS administration. Results are presented in Figure 10.

### Elimination of Free PGE₁-Induced Mortality

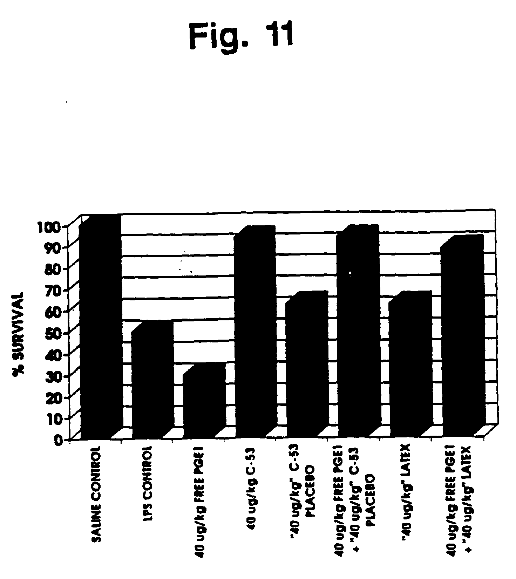

Male Sprague-Dawley rats were injected i.v. with 50 µg/kg LPS at time 0. Free PGE₁ (40µg/kg), LUV-PGE₁ (40 µg/kg PGE₁), placebo LUVs (40 µg/kg lipid equivalency, i.e., the number of placebo LUVs was equal to the number of LUVs present in connection with a dose of 40 micrograms of prostaglandin E1 per kg of body weight), latex microspheres (the number equivalent to the number of placebo LUVs) or latex microspheres plus free PGE₁ simultaneously injected i.v. Survival in treatment group (16 rats) was asset 24 hours. Results are presented Figure 11.

## Claims

1. A method for the manufacture of a pharmaceutical composition for the treatment of a disorder characterized by cell activation and adhesion, inflammation or toxemia comprising formulating:
(a) a pharmaceutically acceptable carrier; and,
(b) a unilammellar liposome comprising:
(i) a lipid;
(ii) a release-inhibiting aqueous buffer
(iii) an anti-disorder effective amount of an arachidonic acid metabolite which is at least about 10⁻¹² g of the metabolite per kg of body weight of the animal to be treated, wherein said release-inhibiting buffer increases the strength of metabolite-lipid interactions or establishes electrostatic repulsions with the metabolite.

2. The method of claim 1, which is suitable for intravenous administration.

3. The method of claim 1, wherein the unilamellar liposome is a large unilamellar liposome having a diameter of about 100 nm.

4. The method of claim 1, wherein the arachidonic acid metabolite is a prostaglandin.

5. The method of claim 4, wherein the prostaglandin is prostaglandin E1.

6. The method of claim 1, wherein the lipid is a saturated acyl-chain lipid.

7. The method of claim 6, wherein the saturated acyl chain lipid is dipalmitoyl phosphatidylcholine.

8. The method of claim 1, wherein the buffer is a citric acid buffer.

9. The method of claim 8, wherein the citric acid buffer has a pH of about 4.5.

10. The method of claim 1, wherein the disorder comprises reperfusion injury, systemic inflammatory response syndrome, myocardial infarction, adult respiratory distress syndrome, vasculitis, burn injury, post-traumatic shock, a vaso-occlusive disorder, in arthritic disorder or an autoimmune disorder.

11. The method of claim 10, wherein the arthritic disorder is rheumatoid arthritis, gout or filary arthritis.

12. The method of claim 10, wherein the autoimmune disorder is systemic lupus erythematosus, juvenile diabetes, multiple sclerosis or Hashimoto's thyroiditis.

13. The method of claim 10 , wherein the disorder comprises systemic inflammatory response syndrome or adult respiratory distress syndrome.

14. The method of claim 1 , wherein the effective amount of the metabolite is from about 10⁻¹² g of the metabolite per kg of body weight of the animal to about 10⁻³ g per kg.

15. The method of claim 14, wherein the effective amount of the metabolite is from about 10⁻⁸ g of the metabolite per kg of body weight of the animal to about 10⁻⁴ g per kg.

16. The method of claim 15, wherein the effective amount of the metabolite is about 10⁻⁶ g of the metabolite per kg of the body weight of the animal.

17. The method of claim 1, wherein the liposome comprises a drying protectant.

18. The method of claim 17, wherein the drying protectant is a saccharide.

19. The method of claim 18, wherein the saccharide is maltose, lactose, dextrose, trehalose, raffinose or sucrose.

20. The method of claim 19, wherein the saccharide is maltose.

21. The method of claim 1, comprising an additional bioactive agent.

22. Use of a composition comprising a pharmaceutically acceptable carrier and a unilamellar liposome comprising an arachidonic acid metabolite, a lipid and a release-inhibiting aqueous buffer wherein said release-inhibiting buffer increases the strength of metabolite-lipid interactions or establishes electrostatic repulsions with the metabolite for the manufacture of a composition for the treatment of an animal afflicted with a disorder characterized by cell activation and adhesion, inflammation or toxemia.

23. The use of claim 22 , wherein the animal is a human.

## Patentansprüche

1. Verfahren zur Herstellung einer pharmazeutischen Zusammensetzung zur Behandlung einer Krankheit, gekennzeichnet durch Zellaktivierung und -Adhäsion, Entzündung oder Toxämie, umfassend das Formulieren:
(a) eines pharmazeutisch verträglichen Trägers; und
(b) eines unilamellaren Liposoms, umfassend:
(i) ein Lipid;
(ii) einen freisetzungsinhibierenden wäßrigen Puffer;
(iii) eine gegen die Krankheit wirksame Menge eines Arachidonsäuremetaboliten, die mindestens etwa 10⁻¹² g des Metaboliten pro kg Körpergewicht des zu behandelnden Lebewesens ist, worin der freisetzungsinhibierende Puffer die Stärke der Metabolit-Lipid-Wechselwirkungen erhöht oder elektrostatische Abstoßungen mit dem Metaboliten bewirkt.

2. Verfahren nach Anspruch 1, das zur intravenösen Verabreichung geeignet ist.

3. Verfahren nach Anspruch 1, worin das unilamellare Liposom ein großes unilamellares Liposom mit einem Durchmesser von etwa 100 nm ist.

4. Verfahren nach Anspruch 1, worin der Arachidonsäuremetabolit ein Prostaglandin ist.

5. Verfahren nach Anspruch 4, worin das Prostaglandin Prostaglandin E1 ist.

6. Verfahren nach Anspruch 1, worin das Lipid ein Lipid mit gesättigter Acylkette ist.

7. Verfahren nach Anspruch 6, worin das Lipid mit gesättigter Acylkette Dipalmitoylphosphatidylcholin ist.

8. Verfahren nach Anspruch 1, worin der Puffer ein Zitronensäurepuffer ist.

9. Verfahren nach Anspruch 8, worin der Zitronensäurepuffer einen pH von etwa 4,5 hat.

10. Verfahren nach Anspruch 1, worin die Krankheit eine Reperfusionsverletzung, ein systemisches Endzündungsreaktionssyndrom, einen Myocardialinfarkt, ein adultes Atemnotssyndrom, eine Vaskulitis, eine Brandverletzung, einen posttraumatischen Schock, eine vasookklusive Erkrankung, eine arthritische Erkrankung oder eine Autoimmunerkrankung umfaßt.

11. Verfahren nach Anspruch 10, worin die arthritische Erkrankung rheumatoide Arthritis, Gicht oder Filararthritis ist.

12. Verfahren nach Anspruch 10, worin die Autoimmunerkrankung systemischer Lupus Erythematodes, juveniler Diabetes, multiple Sklerose oder Hashimoto-Thyreoiditis ist.

13. Verfahren nach Anspruch 10, worin die Erkrankung das systemische Entzündungsreaktionssyndrom oder das adulte Atemnotssyndrom umfaßt.

14. Verfahren nach Anspruch 1, worin die wirksame Menge des Metaboliten von etwa 10⁻¹² g des Metaboliten pro kg Körpergewicht des Lebewesens bis etwa 10⁻³ g pro kg ist.

15. Verfahren nach Anspruch 14, worin die wirksame Menge des Metaboliten von etwa 10⁻⁸ g des Metaboliten pro kg Körpergewicht des Lebewesens bis etwa 10⁻⁴ g pro kg ist.

16. Verfahren nach Anspruch 15, worin die wirksame Menge des Metaboliten etwa 10⁻⁶ g des Metaboliten pro kg Körpergewicht des Lebewesens ist.

17. Verfahren nach Anspruch 1, worin das Liposom ein Trocknungsschutzmittel umfaßt.

18. Verfahren nach Anspruch 17, worin das Trocknungsschutzmittel ein Saccharid ist.

19. Verfahren nach Anspruch 18, worin das Saccharid Maltose, Lactose, Dextrose, Trehalose, Raffinose oder Saccharose ist.

20. Verfahren nach Anspruch 19, worin das Saccharid Maltose ist.

21. Verfahren nach Anspruch 1, umfassend ein zusätzliches bioaktives Mittel.

22. Verwendung einer Zusammensetzung, die einen pharmazeutisch verträglichen Träger und ein unilamellares Liposom, umfassend einen Arachidonsäuremetaboliten, ein Lipid und einen freisetzungsinhibierenden wäßrigen Puffer enthält, worin der freisetzungsinhibierende Puffer die Stärke der Metabolit-Lipid-Wechselwirkungen erhöht oder elektrostatische Abstoßungen mit dem Metaboliten bewirkt, zur Herstellung einer Zusammensetzung zur Behandlung eines Lebewesens, das von einer Krankheit befallen ist, die durch eine Zellaktivierung und -Adhäsion, Entzündung oder Toxämie gekennzeichnet ist.

23. Verwendung nach Anspruch 22, worin das Lebewesen ein Mensch ist.

## Revendications

1. Procédé pour la préparation d'une composition pharmaceutique destinée au traitement d'un trouble caractérisé par une activation et une adhésion cellulaires, une inflammation ou une toxémie, comprenant la formulation d'une composition comprenant:
(a) un véhicule pharmaceutiquement acceptable et
(b) un liposome unilamellaire comprenant
(I) un lipide,
(II) un tampon aqueux inhibiteur de libération et
(III) une quantité anti-trouble efficace d'un métabolite d'acide arachidonique, qui est d'au moins environ 10⁻¹² g du métabolite par kg de poids corporel de l'animal à traiter,
ledit tampon inhibiteur de libération augmentant la force d'interactions métabolite-lipide ou établissant des répulsions électrostatiques avec le métabolite.

2. Procédé de la revendication 1, qui est approprié à l'administration intraveineuse.

3. Procédé de la revendication 1, dans lequel le liposome unilamellaire est un liposome unilamellaire de grande taille, ayant un diamètre d'environ 100 nm.

4. Procédé de la revendication 1, dans lequel le métabolite d'acide arachidonique est une prostaglandine.

5. Procédé de la revendication 4, dans lequel la prostaglandine est la prostaglandine E₁.

6. Procédé de la revendication 1, dans lequel le lipide est un lipide à chaîne acyle saturée.

7. Procédé de la revendication 6, dans lequel le lipide à chaîne acyle saturée est la dipalmitoyl-phosphatidylcholine.

8. Procédé de la revendication 1, dans lequel le tampon est un tampon acide citrique.

9. Procédé de la revendication 8, dans lequel le tampon acide citrique a un pH d'environ 4,5.

10. Procédé de la revendication 1, dans lequel le trouble comprend un trouble de reperfusion, le syndrome de réponse inflammatoire systémique, l'infarctus du myocarde, le syndrome de détresse respiratoire de l'adulte, l'angéite, une lésion due à une brûlure, un choc post-traumatique, un trouble vaso-occlusif, une affection arthritique ou une maladie auto-immune.

11. Procédé de la revendication 10, dans lequel l'affection arthritique est la polyarthrite rhumatoïde, la goutte ou l'arthrite à filaires.

12. Procédé de la revendication 10, dans lequel la maladie auto-immune est le lupus érythémateux disséminé, le diabète juvénile, la sclérose en plaques ou la thyroïdite de Hashimoto.

13. Procédé de la revendication 10, dans lequel le trouble comprend le syndrome de réponse inflmammatoire systémique ou le syndrome de détresse respiratoire de l'adulte.

14. Procédé de la revendication 1, dans lequel la quantité efficace du métabolite va d'environ 10⁻¹² g du métabolite par kg de poids corporel de l'animal à environ 10⁻³ g par kg.

15. Procédé de la revendication 14, dans lequel la quantité efficace du métabolite va d'environ 10⁻⁸ g du métabolite par kg de poids corporel de l'animal à environ 10⁻⁴ g par kg.

16. Procédé de la revendication 15, dans lequel la quantité efficace du métabolite est d'environ 10⁻⁶ g du métabolite par kg de poids corporel de l'animal.

17. Procédé de la revendication 1, dans lequel le liposome comprend un agent protecteur lors de la dessication.

18. Procédé de la revendication 17, dans lequel l'agent protecteur lors de la dessication est un saccharide.

19. Procédé de la revendication 18, dans lequel le saccharide est le maltose, le lactose, le D-glucose, le tréhalose, le raffinose ou le saccharose.

20. Procédé de la revendication 19, dans lequel le saccharide est le maltose.

21. Procédé de la revendication 1, comprenant un agent bioactif supplémentaire.

22. Utilisation d'une composition comprenant un véhicule pharmaceutiquement acceptable et un liposome unilamellaire comprenant un métabolite d'acide arachidonique, un lipide et un tampon aqueux inhibiteur de libération, ledit tampon inhibiteur de libération augmentant la force d'interactions métabolite-lipide ou établissant des répulsions électrostatiques avec le métabolite, pour la préparation d'une composition destinée au traitement d'un animal atteint d'un trouble caractérisé par une activation et une adhésion cellulaires, une inflammation ou une toxémie.

23. Utilisation selon la revendication 22, dans laquelle l'animal est un sujet humain.
